(19) Europäisches Patentamt
European Patent Office
Office européen des brevets

(11) EP 4 692 317 A1

(12) **EUROPEAN PATENT APPLICATION**
published in accordance with Art. 153(4) EPC

(43) Date of publication:
**11.02.2026 Bulletin 2026/07**

(21) Application number: **25792066.0**

(22) Date of filing: **23.05.2025**

(51) International Patent Classification (IPC):
***C12N 5/00*** *(2006.01)* ***C12M 1/12*** *(2006.01)*
***C08F 212/08*** *(2006.01)* ***C08F 212/12*** *(2006.01)*

(52) Cooperative Patent Classification (CPC):
**C12M 1/12; C12N 5/00**

(86) International application number:
**PCT/KR2025/095362**

(87) International publication number:
**WO 2025/244512 (27.11.2025 Gazette 2025/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **23.05.2024 KR 20240067364**

(71) Applicant: **LG Chem, Ltd.**
**Seoul 07336 (KR)**

(72) Inventors:
- **KIM, Yeji**
  **Daejeon 34122 (KR)**
- **KIM, Minchae**
  **Daejeon 34122 (KR)**
- **KIM, Jee Seon**
  **Daejeon 34122 (KR)**

(74) Representative: **Plasseraud IP**
**104 Rue de Richelieu**
**CS92104**
**75080 Paris Cedex 02 (FR)**

# (54) MICROCARRIER FOR CELL CULTURE, METHOD FOR PREPARING MICROCARRIER FOR CELL CULTURE, AND CELL CULTURE COMPOSITION USING SAME

(57) The present disclosure relates to a microcarrier for cell culture comprising polystyrene-based particles, a preparing method for the microcarrier for cell culture, and a cell culture composition using the same.

**FIG. 1**

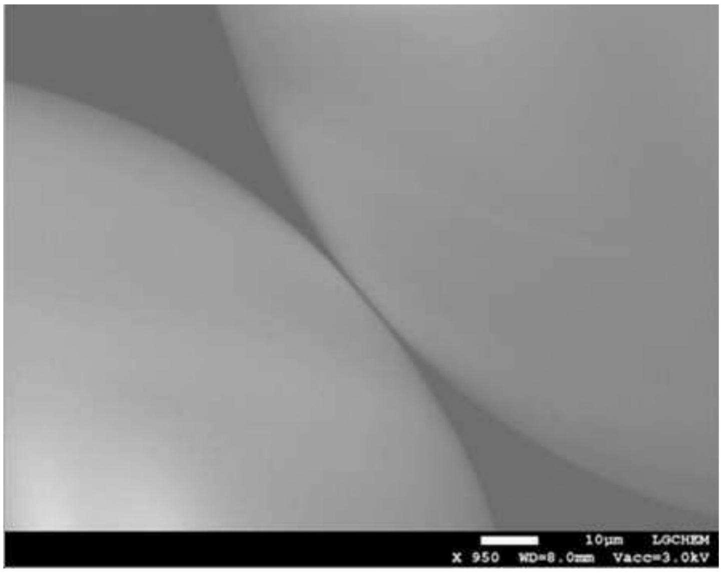


EP 4 692 317 A1

## Description

### [TECHNICAL FIELD]

This application claims the benefit of priority based on Korean Patent Application No. 10-2024-0067364 filed on May 23, 2024, and Korean Patent Application No. 10-2025-0067201 filed on May 23, 2025, the disclosures of which are incorporated herein by reference in their entirety.

The present disclosure relates to a microcarrier for cell culture, a preparing method for microcarrier for cell culture, and a cell culture composition using the same.

### [BACKGROUND]

As the biopharmaceutical and regenerative medical fields expand, there is a growing demand for large-scale cell culture technology capable of efficiently producing cells, tissues, microorganisms, and the like.

Adherent cells are cultured using micro carriers in a 3D bioreactor. The cells, culture medium and microcarrier are placed in the bioreactor, and the culture medium is agitated to bring the cells into contact with the micro carriers, so that the cells are adhered onto the surface of the micro carrier and cultured. Since the micro carrier used at this time provides a high surface area/volume ratio to which cells may attach and grow com pared to 2D cultures, it is suitable for large-scale culture of cells.

Currently commercially available microcarriers have a density of about 1.1 to 1.3 g/$cm^3$, and cells have a density of about 1.2 g/$cm^3$. In this case, it is advantageous for attaching cells at the initial stage of culture in the bioreactor, but it is difficult to perform centrifugation when separating and recovering cells after culture, and a filtering method based on the microcarrier and cell size should be used. However, in this case, there are problems that the filter is clogged or the process requires a long time, and physical damage and contamination of cells may easily occur, and loss of cells may occur.

To address these issues, microcarriers have been fabricated from materials with densities lower than 1.0 g/$cm^3$ or higher than 1.3 g/$cm^3$. However, the attainable density range is limited, and it is difficult to achieve a sufficient yield of perfectly spherical microcarriers that are free from damage or breakage.

### [DETAILED DESCRIPTION OF THE INVENTION]

### [Technical Problem]

The present disclosure relates to a microcarrier for cell culture whose density is controlled so that it may be separated from cells by a density difference, while simultaneously exhibiting improved dispersibility in cell culture reactors or media and enhanced cell adhesion.

The present disclosure also relates to a preparing method for microcarrier for cell culture.

The present disclosure also relates to a cell culture composition using the microcarrier for cell culture.

### [Technical Solution]

According to an embodiment of the present disclosure, there is provided a microcarrier for cell culture comprising polystyrene-based particles that include a monomer compound represented by Chemical Formula 1 below:

[Chemical Formula 1]

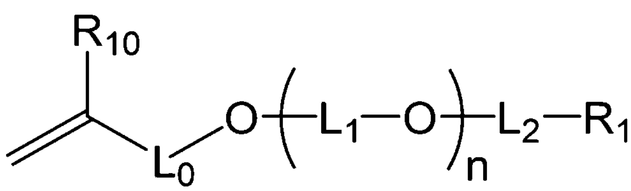

wherein in Chemical Formula 1, $L_0$ is an arylene group having 6 or more carbon atoms or -(C=O)-, $L_1$ and $L_2$ are each independently an alkylene group having 1 or more carbon atoms, $R_1$ is a reactive functional group capable of ring-opening reaction, $R_{10}$ is hydrogen or an alkyl group having 1 or more carbon atoms, and n is an integer of 0 or greater.

According to another embodiment of the present disclosure, there is provided a preparing method for microcarrier for cell culture, comprising the step of: polymerizing and recovering polystyrene-based particles from a monomer mixture including a compound represented by the following Chemical Formula 1 below:

[Chemical Formula 1]

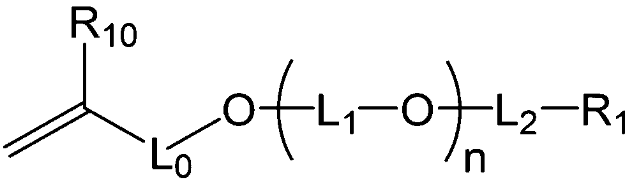

wherein in Chemical Formula 1, $L_0$ is an arylene group having 6 or more carbon atoms or -(C=O)-, $L_1$ and $L_2$ are each independently an alkylene group having 1 or more carbon atoms, $R_1$ is a reactive functional group capable of ring-opening reaction, $R_{10}$ is hydrogen or an alkyl group having 1 or more carbon atoms, and n is an integer of 0 or greater.

According to yet another embodiment of the present disclosure, there is provided a cell-culture composition comprising cells and the above microcarrier for cell culture.

Now, a microcarrier for cell culture, a preparing method for microcarrier for cell culture, and a cell culture composition using the same according to specific embodiments of the present disclosure will be described in more detail.

Unless particularly mentioned herein, technical terms are used only to refer to particular embodiments and are not intended to limit the present disclosure.

Singular forms used herein include plural forms unless the context clearly indicates otherwise.

The term "including" or "comprising" as used herein specifies the presence of the stated features, regions, integers, steps, operations, elements, and/or components, but does not exclude the presence or addition of one or more other features, regions, integers, steps, operations, elements, components, and/or groups.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present disclosure.

As used herein, an alkyl group may be straight-chain or branched; although the number of carbon atoms is not particularly limited, it is preferably 1 to 10. In another embodiment, the alkyl group contains 1 to 6 carbon atoms. Specific examples include, without limitation, methyl, ethyl, propyl, n-propyl, isopropyl, butyl, n-butyl, isobutyl, tert-butyl, sec-butyl, 1-methylbutyl, 1-ethylbutyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethylpropyl, 1,1-dimethylpropyl, isohexyl, 4-methylhexyl, and 5-methylhexyl.

As used herein, a cycloalkyl group is a monovalent substituent derived from a cycloalkane and may be monocyclic or polycyclic; although not particularly limited, it preferably has 3 to 20 carbon atoms. In another embodiment, the cycloalkyl group has 3 to 10 carbon atoms. Specific examples include, without limitation, cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and bicyclo[2.2.1]heptyl. The cycloalkyl group may be substituted or unsubstituted, and examples of substituents are as described above.

As used herein, a heterocycloalkyl group refers to a cycloalkyl group containing at least one heteroatom other than carbon; specific heteroatoms may include one or more selected from O, N, Se, and S.

The disclosure will be described in more detail.

According to an embodiment of the present disclosure, there may be provided a a microcarrier for cell culture comprising polystyrene-based particles that include a monomer compound represented by the Chemical Formula 1.

The present inventors have found through experiments that in the case of the microcarrier for cell culture of this embodiment, including the compound represented by the Chemical Formula 1 as a monomer of the polystyrene-based polymer makes it possible to control the density of the final microcarrier for cell culture, allowing separation based on the density difference from cells, while simultaneously improving dispersibility in cell culture reactors or media and enhancing cell adhesion, and completed the present disclosure.

Conventional microcarriers for cell culture controlled the density of particles by encapsulating non-reactive low-density oil inside the particles, but there was a technical problem in that the low-density oil encapsulated inside leaked out when the particles were physically damaged.

Accordingly, the inventors confirmed that, by including the compound represented by the Chemical Formula 1 as a monomer in the polystyrene-based polymer used for the microcarrier for cell culture, particle density may be regulated, enabling separation based on the density difference from cells, and simultaneously improving dispersibility in a bioreactor or culture medium.

In addition, conventional microcarriers for cell culture formed a coating layer on the surface of polystyrene particles to enhance cell adhesion, but there was a technical problem in that the part of the coating layer may be detached

when the microcarriers are agitated in the culture medium.

Therefore, the inventors verified that by including the compound represented by the Chemical Formula 1 as a monomer in the polystyrene-based polymer used for the microcarrier for cell culture, reactive sites allowing cell-adhesive ligands to be chemically immobilized without an additional coating layer on the microcarrier surface may be provided thereby improving cell adhesion.

Specifically, in one embodiment of the microcarrier for cell culture, the microcarrier may comprise polystyrene-based particles including a monomer compound represented by Chemical Formula 1 below:

[Chemical Formula 1]

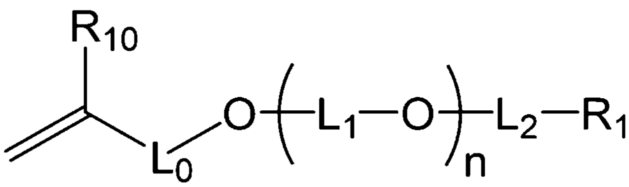

wherein in Chemical Formula 1, $L_0$ is an arylene group having 6 or more carbon atoms or -(C=O)-, $L_1$ and $L_2$ are each independently an alkylene group having 1 or more carbon atoms, $R_1$ is a reactive functional group capable of ring-opening reaction, $R_{10}$ is hydrogen or an alkyl group having 1 or more carbon atoms, and n is an integer of 0 or greater.

As the compound represented by Chemical Formula 1 includes a reactive functional group capable of a ring-opening reaction, reactive sites allowing cell-adhesive ligands to be chemically immobilized without an additional coating layer on the microcarrier surface may be provided thereby improving cell adhesion.

The reactive functional group capable of a ring-opening reaction refers to a functional group that may participate in ring-opening processes such as hydrolyzed ring opening.

Specifically, the reactive functional group capable of ring-opening reaction may include a heterocycloalkyl group.

The heterocycloalkyl group may be a cycloalkyl group including one or more non-carbon atoms, i.e., heteroatoms. The heteroatoms may include at least one atom selected from the group consisting of O, N, Se, and S, preferably O.

The heterocycloalkyl group is not particularly limited, but, for example, may include an epoxy group.

Specifically, the polystyrene-based particles may include a reaction product of the compound represented by Chemical Formula 1 and an ethylene-based unsaturated crosslinking agent, and may include 1 part by weight or more and 15 parts by weight or less of the compound represented by Chemical Formula 1, based on 100 parts by weight of the ethylene-based unsaturated crosslinking agent.

More specifically, the polystyrene-based particles may include a reaction product of the compound represented by Chemical Formula 1 and an ethylene-based unsaturated crosslinking agent, and, based on 100 parts by weight of the ethylene-based unsaturated crosslinking agent, may contain 1 part by weight or more, 1.5 parts by weight or more, 15 parts by weight or less, 10 parts by weight or less, 5 parts by weight or less, or 1 part by weight or more and 15 parts by weight or less, 1.5 parts by weight or more and 15 parts by weight or less, 1 part by weight or more and 10 parts by weight or less, 1.5 parts by weight or more and 10 parts by weight or less, 1 part by weight or more and 5 parts by weight or less, or 1.5 parts by weight or more and 5 parts by weight or less of the compound represented by Chemical Formula 1.

When the compound represented by Chemical Formula 1 is included in an excessively small amount with respect to 100 parts by weight of ethylene-based unsaturated crosslinking agent, an insufficient number of reaction sites capable of immobilizing the cell-adhesive ligand is provided, potentially resulting in poor cell adhesiveness. Conversely, when the compound represented by Chemical Formula 1 is included in an excessively large amount, the relative proportion of the high-density compound represented by Chemical Formula 1 increases, so that the particle density becomes higher than that of the cell culture medium, leading to a loss of low-density characteristics.

Specifically, the compound represented by Chemical Formula 1 may include at least one compound selected from the group consisting of the compounds represented by Chemical Formulas 1-1 to 1-3 below.

[Chemical Formula 1-1]

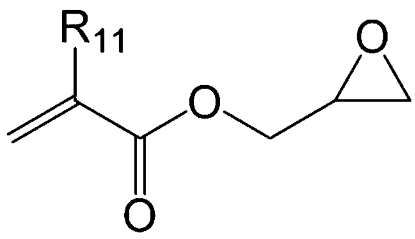

wherein in Chemical Formula 1-1, $R_{11}$ is hydrogen or an alkyl group having one or more carbon atoms,

[Chemical Formula 1-2]

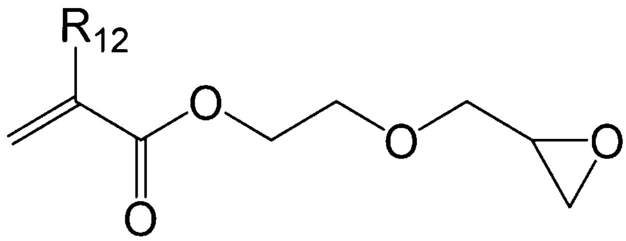

wherein in Chemical Formula 1-2, $R_{12}$ is hydrogen or an alkyl group having at least one carbon atom,

[Chemical Formula 1-3]

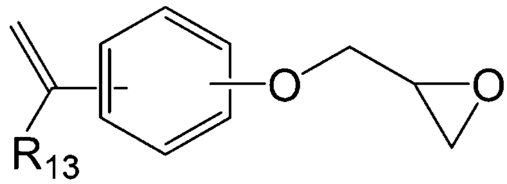

wherein in Chemical Formula 1-3, $R_{13}$ is hydrogen or an alkyl group having one or more carbon atoms.

By including at least one compound selected from the group consisting of the compounds represented by Chemical Formulas 1-1 to 1-3 as a monomer of the polystyrene-based polymer, a reaction site capable of immobilizing a cell-adhesive ligand on the surface of a microcarrier for cell culture through chemical bonding may be provided without an additional coating layer, by adjusting the weight ratio between the styrene-based monomer and the compounds represented by Chemical Formulas 1-1 to 1-3, thereby improving cell adhesiveness.

Meanwhile, the polystyrene-based particles may further include, as a monomer compound, a compound represented by the following Chemical Formula 2 in addition to the compound represented by Chemical Formula 1:

[Chemical Formula 2]

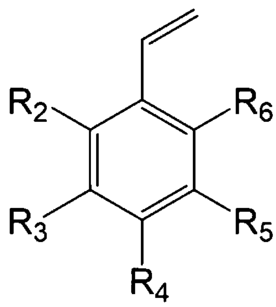

wherein in Chemical Formula 2, $R_2$ to $R_6$ are each independently hydrogen or an alkyl group having one or more carbon atoms, and at least one of $R_2$ to $R_6$ is an alkyl group having one or more carbon atoms.

By including the compound represented by the following Chemical Formula 2 as a monomer of the polystyrene-based particles, the density of the microcarrier for cell culture may be precisely controlled by adjusting the content of the compound represented by Chemical Formula 2, thereby enabling separation based on the density difference from cells, while simultaneously improving dispersibility in the cell-culture reactor or medium.

Specifically, the compound represented by Chemical Formula 2 may include at least one compound selected from the group consisting of the compounds represented by Chemical Formulas 2-1 to 2-3 below.

[Chemical Formula 2-1]

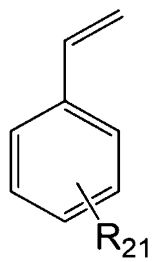

[Chemical Formula 2-2]

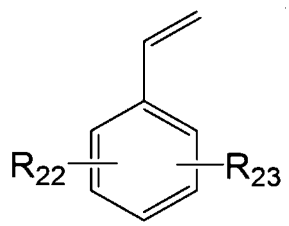

[Chemical Formula 2-3]

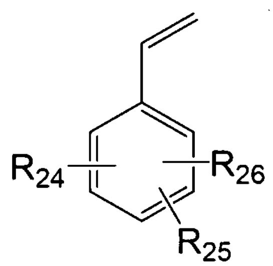

wherein in Chemical Formulas 2-1 to 2-3, $R_{21}$ to $R_{26}$ are each independently an alkyl group having one or more carbon atoms.

By including at least one compound selected from the group consisting of the compounds represented by Chemical Formulas 2-1 to 2-3 as a monomer of the polystyrene-based particles, the density of the microcarrier for cell culture may be precisely controlled by adjusting the content of the compound represented by Chemical formula 2, thereby enabling separation based on the density difference from cells and simultaneously improving dispersibility in a cell-culture reactor or medium.

More specifically, the compound represented by Chemical formula 2 may include at least one compound selected from the group consisting of the compound represented by the following Chemical Formulas 2-4 to 2-6:

[Chemical Formula 2-4]

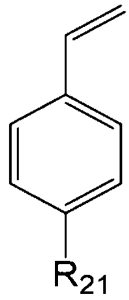

[Chemical Formula 2-5]

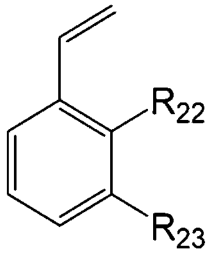

[Chemical Formula 2-6]

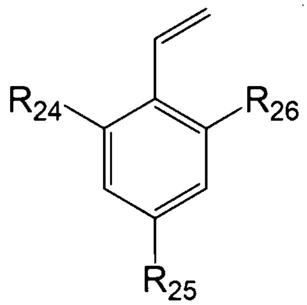

wherein in Chemical Formulas 2-4 to 2-6, $R_{21}$ to $R_{26}$ are each independently an alkyl group having one or more carbon atoms.

Meanwhile, the compound represented by Chemical Formula 2 may have a density of 0.92 g/$cm^3$ or less.

Specifically, the compound represented by Chemical Formula 2 may have a density of 0.92 g/$cm^3$ or less, 0.91 g/$cm^3$ or less, 0.906 g/$cm^3$ or less, 0.9 g/$cm^3$ or less, 0.89 g/$cm^3$ or less, 0.5 g/$cm^3$ or more, 0.6 g/$cm^3$ or more, 0.7 g/$cm^3$ or more, 0.8 g/$cm^3$ or more, 0.5 g/$cm^3$ or more and 0.92 g/$cm^3$ or less, 0.6 g/$cm^3$ or more and 0.92 g/$cm^3$ or less, 0.7 g/$cm^3$ or more and 0.92 g/$cm^3$ or less, 0.8 g/$cm^3$ or more and 0.92 g/$cm^3$ or less, 0.5 g/$cm^3$ or more and 0.91 g/$cm^3$ or less, 0.6 g/$cm^3$ or more and 0.91 g/$cm^3$ or less, 0.7 g/$cm^3$ or more and 0.91 g/$cm^3$ or less, 0.8 g/$cm^3$ or more and 0.91 g/$cm^3$ or less, 0.5 g/$cm^3$ or more and 0.906 g/$cm^3$ or less, 0.6 g/$cm^3$ or more and 0.906 g/$cm^3$ or less, 0.7 g/$cm^3$ or more and 0.906 g/$cm^3$ or less, 0.8 g/$cm^3$ or more and 0.906 g/$cm^3$ or less, 0.5 g/$cm^3$ or more and 0.9 g/$cm^3$ or less, 0.6 g/$cm^3$ or more and 0.9 g/$cm^3$ or less, 0.7 g/$cm^3$ or more and 0.9 g/$cm^3$ or less, 0.8 g/$cm^3$ or more and 0.9 g/$cm^3$ or less, 0.5 g/$cm^3$ or more and 0.89 g/$cm^3$ or less, 0.6 g/$cm^3$ or more and 0.89 g/$cm^3$ or less, 0.7 g/$cm^3$ or more and 0.89 g/$cm^3$ or less, 0.8 g/$cm^3$ or more and 0.89 g/$cm^3$ or less.

By the compound represented by Chemical Formula 2 having a density of 0.92 g/$cm^3$ or less, the density of the microcarrier for cell culture may be precisely controlled by adjusting the content of the compound represented by Chemical Formula 2, enabling separation based on the density difference from cells, while simultaneously improving dispersibility in a cell-culture bioreactor or medium.

For example, the compound represented by Chemical Formula 2 may include at least one compound selected from the group consisting of 4-methylstyrene, trimethylstyrene, 4-ethenyl-2-methyl-1-(2-methylpropyl)benzene, 1-ethenyl-2-methyl-4-(1-methylethyl)benzene, 1-ethenyl-2,3-dimethylbenzene, 4-(1,1-dimethylethyl)-2-ethenyl-1-methylbenzene, 2-ethenyl-4-methyl-1-(1-methylethyl)benzene, 1-ethenyl-2-(1-methylethyl)benzene, 2-tert-butylstyrene, 4-tert-butylstyrene, and 1-ethenyl-3-(1-methylethyl)benzene.

Meanwhile, the polystyrene-based particles may further include, as a monomer, a compound represented by Chemical Formula 3 in addition to the compound represented by Chemical Formula 1.

[Chemical Formula 3]

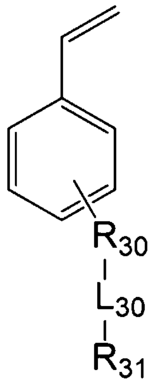

wherein in Chemical Formula 3, $L_{30}$ is -O(C=O)- or -(C=O)O-, $R_{30}$ is a direct bond or an alkylene group having one or more carbon atoms, and $R_{31}$ is an alkyl group having one or more carbon atoms.

That is, the polystyrene-based particles may include, as monomer components, the compound represented by Chemical Formula 1; or the compounds represented by Chemical Formula 1 and Chemical Formula 2; or the compounds represented by Chemical Formula 1 and Chemical Formula 3; or the compounds represented by Chemical Formula 1, Chemical Formula 2, and Chemical Formula 3.

By incorporating the compound represented by Chemical Formula 3 as a monomer of polystyrene-based particles, the substituent of the finally produced microcarrier for cell culture may be hydrolyzed, thereby improving dispersibility in a cell culture reactor or medium.

For example, the compound represented by Chemical Formula 3 may include acetoxystyrene.

Meanwhile, the microcarrier for cell culture may include polystyrene-based particles. Preferably, the microcarrier for cell culture may be consisted of polystyrene-based particles.

Specifically, the polystyrene-based particles may have the apparent density in a range of 0.99 $g/cm^3$ to 1.04 $g/cm^3$. By maintaining this low-density range, the cells and the microcarrier may be readily separated after culture through the difference in sedimentation velocity under gravity when the microcarrier and cells are recovered.

When the density of the polystyrene-based particles exceeds 1.04 $g/cm^3$, the density difference between the cells and the microcarrier becomes small, which may make centrifugation difficult when separating and recovering the cells after culture. Conversely, when the density is less than 0.99 $g/cm^3$, the microcarrier may float only on the surface of the culture medium at the initial stage, making it difficult for cells to adhere.

The cells are adherent animal cells, without particular limitation thereto, and may include, for example, at least one cell selected from the group consisting of the fibroblasts, epithelial cells, osteoblasts, chondrocytes, hepatocytes, human umbilical cord blood-derived cells, human bone marrow-derived mesenchymal stem cells, CHO (Chinese hamster ovary) cells, kidney cells (HEK293, BHK21, MDCK, Vero cells, etc.), or a mixture of two or more thereof.

In addition, the density difference between the microcarrier for cell culture and the cells may be 0.20 $g/cm^3$ or less. Satisfying a density difference of 0.20 $g/cm^3$ or less allows the cells and the microcarrier to be easily separated after culture by exploiting the difference in sedimentation velocity under gravity when they are recovered, while also preventing the problem in which, at the early stage of culture, the microcarrier floats only on the surface of the culture medium, making cell attachment difficult.

The polystyrene-based particles may include the reaction product of a monomer mixture and an ethylene-based unsaturated crosslinking agent.

As described above, the monomer mixture may include the compound represented by Chemical Formula 1. The monomer mixture may also include the compound represented by Chemical Formula 1 and the compound represented by Chemical Formula 2. The monomer mixture may further include the compound represented by Chemical Formula 1 and the compound represented by Chemical Formula 3. In addition, the monomer mixture may include the compounds represented by Chemical Formulas 1 to 3.

That is, the polystyrene-based particles may include the reaction product of the compound represented by Chemical Formula 1, a styrene-based monomer mixture, and an ethylene-based unsaturated crosslinking agent, and the styrene-based monomer mixture may include the compound represented by Chemical Formula 2 or the compound represented by Chemical Formula 3.

Specifically, based on 100 parts by weight of the ethylene-based unsaturated crosslinking agent, the compound represented by Chemical Formula 2 may be included in an amount of 80 parts by weight or more and 150 parts by weight or less.

More specifically, based on 100 parts by weight of the ethylene-based unsaturated crosslinking agent, the compound represented by Chemical Formula 2 may be included in an amount of 80 parts by weight or more, 85 parts by weight or more, 90 parts by weight or more, 150 parts by weight or less, 125 parts by weight or less, 100 parts by weight or less, 99 parts by weight or less, 80 parts by weight or more and 150 parts by weight or less, 85 parts by weight or more and 150 parts by weight or less, 90 parts by weight or more and 150 parts by weight or less, 80 parts by weight or more and 125 parts by weight or less, 85 parts by weight or more and 125 parts by weight or less, 90 parts by weight or more and 125 parts by weight or less, 80 parts by weight or more and 100 parts by weight or less, 85 parts by weight or more and 100 parts by weight or less, 90 parts by weight or more and 100 parts by weight or less, 80 parts by weight or more and 99 parts by weight or less, 85 parts by weight or more and 99 parts by weight or less, 90 parts by weight or more and 99 parts by weight or less.

By including the compound represented by Chemical Formula 2 in an amount of 80 parts by weight or more and 150 parts by weight or less base on 100 parts by weight of the ethylene-based unsaturated crosslinking agent, the low-density characteristics of the particles may be controlled due to the low density of the compound represented by the chemical formula 2, enabling separation by density difference from cells, and at the same time, dispersibility in a cell culture reactor or medium may be improved.

When the compound represented by Chemical Formula 2 is included in too small an amount relative to 100 parts by weight of the ethylene-based unsaturated crosslinker, particle density increases, causing loss of low-density characteristics and resulting in sedimentation in the medium. Conversely, when it is included in an excessively large amount, particle density falls to below 0.99 g/cm$^3$, requiring high-speed agitation to disperse the particles in the medium, which may adversely affect cell characteristics.

Additionally, based on 100 parts by weight of the styrene-based monomer mixture, the compound represented by Chemical Formula 2 may be included in an amount of 90 parts by weight or more and 100 parts by weight or less.

Specifically, based on 100 parts by weight of the styrene-based monomer mixture, the compound represented by Chemical Formula 2 may be included in an amount of 90 parts by weight or more, 91 parts by weight or more, 100 parts by weight or less, 95 parts by weight or less, or 90 parts by weight or more and 100 parts by weight or less, 91 parts by weight or more and 100 parts by weight or less, 90 parts by weight or more and 95 parts by weight or less, 91 parts by weight or more and 95 parts by weight or less.

By including the compound represented by Chemical Formula 2 in the above-mentioned amount with respect to 100 parts by weight of the styrene-based monomer mixture, the density of the microcarrier for cell culture may be precisely controlled, enabling separation based on the density difference from the cells while simultaneously improving dispersibility in a cell-culture bioreactor or medium.

Additionally, based on 100 parts by weight of the compound represented by Chemical Formula 2, the compound represented by Chemical Formula 1 may be included in an amount of 1 part by weight or more and 15 parts by weight or less.

Specifically, based on 100 parts by weight of the compound represented by Chemical Formula 2, the compound represented by Chemical Formula 1 may be included in an amount of 1 part by weight or more, 1.5 part by weight or more, 15 parts by weight or less, 12 parts by weight or less, 10 parts by weight or less, 6 parts by weight or less, 1 part by weight or more and 15 parts by weight or less, 1.5 part by weight or more and 15 parts by weight or less, 1 part by weight or more and 12 parts by weight or less, 1.5 part by weight or more and 12 parts by weight or less, 1 part by weight or more and 10 parts by weight or less, 1.5 part by weight or more and 10 parts by weight or less, 1 part by weight or more and 6 parts by weight or less, 1.5 part by weight or more and 6 parts by weight or less.

When the compound represented by Chemical Formula 1 is included in an excessively small amount based on 100 parts by weight of the compound represented by Chemical Formula 2, an insufficient number of reactive sites capable of immobilizing the cell-adhesive ligand will be provided, which may lead to poor cell adhesiveness; conversely, when it is included in an excessively large amount, the density of the particles may increase, resulting in a technical problem where the low-density characteristics are lost.

Additionally, when a monomer compound represented by Chemical Formula 3 is included, the compound represented by Chemical Formula 3 may be included in an amount of 0.1 parts by weight or more and 5 parts by weight or less, based on 100 parts by weight of the ethylene-based unsaturated crosslinking agent.

More specifically, based on 100 parts by weight of the ethylene-based unsaturated crosslinking agent, the compound represented by Chemical Formula 3 may be included in an amount of 0.1 parts by weight or more, 0.5 parts by weight or more, 1 part by weight or more, 5 parts by weight or less, 3 parts by weight or less, 2 parts by weight or less, 0.1 parts by weight or more and 5 parts by weight or less, 0.5 parts by weight or more and 5 parts by weight or less, 1 part by weight or more and 5 parts by weight or less, 0.1 parts by weight or more and 3 parts by weight or less, 0.5 parts by weight or more and 3 parts by weight or less, 1 part by weight or more and 3 parts by weight or less, 0.1 parts by weight or more and 2 parts by weight or less, 0.5 parts by weight or more and 2 parts by weight or less, 1 part by weight or more and 2 parts by

weight or less.

When a monomer compound represented by Chemical Formula 3 is included in an amount of 0.1 parts by weight or more and 5 parts by weight or less, based on 100 parts by weight of the ethylene-based unsaturated crosslinking agent, the dispersibility in a cell culture reactor or medium may be improved by hydrolyzing the substituents of the microcarrier for cell culture.

When the compound represented by Chemical Formula 3 is included in an amount exceeding 5 parts by weight, based on 100 parts by weight of the ethylene-based unsaturated crosslinking agent, particle density may increase, leading to a loss of low-density characteristics.

Additionally, when the monomer compound includes the compound represented by Chemical Formula 3, the compound represented by Chemical Formula 3 may be included in an amount of 0.1 parts by weight or more and 5 parts by weight or less, based on 100 parts by weight of the styrene-based monomer mixture

Specifically, based on 100 parts by weight of the styrene-based monomer mixture, the compound represented by Chemical Formula 3 may be included in an amount of 0.1 parts by weight or more, 0.5 parts by weight or more, 1 part by weight or more, 5 parts by weight or less, 3 parts by weight or less, 2.5 parts by weight or less, 0.1 parts by weight or more and 5 parts by weight or less, 0.5 parts by weight or more and 5 parts by weight or less, 1 part by weight or more and 5 parts by weight or less, 0.1 parts by weight or more and 3 parts by weight or less, 0.5 parts by weight or more and 3 parts by weight or less, 1 part by weight or more and 3 parts by weight or less, 0.1 parts by weight or more and 2.5 parts by weight or less, 0.5 parts by weight or more and 2.5 parts by weight or less, 1 part by weight or more and 2.5 parts by weight or less.

When the monomer compound includes the compound represented by Chemical Formula 3, by including the compound represented by Chemical Formula 3 in an amount of 0.1 parts by weight or more and 5 parts by weight or less based on 100 parts by weight of the styrene-based monomer mixture, the dispersibility in a cell culture reactor or medium may be improved by hydrolyzing the substituents of the microcarrier for cell culture.

Additionally, when the monomer compound contains a compound represented by Chemical Formula 3, the compound represented by Chemical Formula 1 may be included in an amount of 110 parts by weight or more and 500 parts by weight or less, based on 100 parts by weight of the compound represented by Chemical Formula 3.

Specifically, based on 100 parts by weight of the compound represented by Chemical Formula 3, the compound represented by Chemical Formula 1 may be included in an amount of 110 parts by weight or more, 150 parts by weight or more, 500 parts by weight or less, 400 parts by weight or less, 300 parts by weight or less, 200 parts by weight or less, 110 parts by weight or more and 500 parts by weight or less, 110 parts by weight or more and 400 parts by weight or less, 110 parts by weight or more and 300 parts by weight or less, 110 parts by weight or more and 200 parts by weight or less, 150 parts by weight or more and 500 parts by weight or less, 150 parts by weight or more and 400 parts by weight or less, 150 parts by weight or more and 300 parts by weight or less, 150 parts by weight or more and 200 parts by weight or less.

When the monomer compound contains a compound represented by Chemical Formula 3, if the compound represented by Chemical Formula 1 is included in an amount of less than 110 parts by weight based on 100 parts by weight of the compound represented by Chemical Formula 3, the compound represented by Chemical Formula 1 may be lost before polymerization due to its relatively high water solubility and may therefore not be introduced onto the particle surface. Conversely, if it is included in an amount exceeding 500 parts by weight, the particle density may become higher, thereby losing low-density characteristics.

Additionally, the polystyrene-based particles may include the ethylene-based unsaturated crosslinking agent in an amount of 60 parts by weight or more and 200 parts by weight or less, based on 100 parts by weight of the styrene-based monomer mixture.

Specifically, based on 100 parts by weight of the styrene-based monomer mixture, the polystyrene-based particles may include the ethylene-based unsaturated crosslinking agent in an amount of 60 parts by weight or more and 200 parts by weight or less, 60 parts by weight or more and 150 parts by weight or less, 60 parts by weight or more and 130 parts by weight or less, 100 parts by weight or more and 200 parts by weight or less, 100 parts by weight or more and 150 parts by weight or less, 100 parts by weight or more and 130 parts by weight or less.

When the ethylene-based unsaturated crosslinking agent is included in an excessively small amount of less than 60 parts by weight based on 100 parts by weight of the styrene-based monomer mixture, the crosslink density of the polystyrene-based polymer decreases, making it difficult to maintain a stable spherical shape of the particles.

Conversely, when the ethylene-based unsaturated crosslinking agent is included in an excessively large amount exceeding 200 parts by weight based on 100 parts by weight of the styrene-based monomer mixture, it becomes difficult to lower the particle density to the desired level.

Examples of the ethylene-based unsaturated crosslinking agent may include divinylbenzene.

The average diameter of the polystyrene-based particles may be 50 μm to 400 μm, or 60 μm to 390 μm. When the average diameter of the polystyrene-based particles falls within the aforementioned range, cell adhesion and culture performance are excellent. When the average diameter is less than 50 μm, the surface area available for cell culture becomes small, which may reduce culture efficiency; when it exceeds 400 μm, interactions among adherent cells decrease and the cell density in the bioreactor is lowered, which may likewise reduce culture efficiency.

The diameter of a polystyrene-based particle refers to the distance between two points where a straight line passing through the particle's center of gravity meets its outermost surface, and the average diameter of the polystyrene-based particles may be determined by measuring, under an optical microscope, the diameters of all the polystyrene-based particles included in the microcarrier for cell culture.

The polystyrene-based particles may be a group of individual particles having an average diameter of 50 μm to 400 μm, or 60 μm to 390 μm, and the individual fine particles included in the group may each have a diameter of, on average, 50 μm to 400 μm, or 60 μm to 390 μm. More specifically, 95 % or more, or 99 % or more of the individual fine particles contained in the group may have a diameter of 50 μm to 400 μm, or 60 μm to 390 μm.

In addition, the polystyrene-based particles may have a percentage of perfectly spherical particles without damage or breakage, as defined by the following equation, of more than 90% and 100% or less, 92% or more and 100% or less, 95% or more and 100% or less, 96% or more and 99% or less.

Percentage of perfectly spherical particles without damage or breakage (%) = (Number of polystyrene-based particles that are perfectly spherical without damage or breakage / Total number of polystyrene-based particles) × 100. [Equation]

The percentage of perfectly spherical particles without damage or breakage determined by the above equation may be obtained for the polystyrene-based particles by counting, via SEM, the number of particles that are perfectly spherical and free of damage or breakage among all particles and then calculating the percentage of those particles relative to the total.

Accordingly, the microcarrier for cell culture may include multiple polystyrene-based particles, and whether each of those particles is perfectly spherical without damage or breakage may be visually confirmed by SEM.

When the proportion of perfectly spherical particles without damage or fracture as defined by the above equation falls to 90% or less, the number of irregular particles with uneven, concave surfaces increases, and these irregular particles remain suspended in the cell culture medium, delivering physical impacts to the cells under cultivation, which may reduce cell culture efficiency to such an extent that successful cell culture becomes impossible.

Specifically, the D50 particle diameter of the microcarrier for cell culture may be in range of 100 μm to 300 μm, 100 μm to 250 μm, 120 μm to 250 μm, or 130 μm to 250 μm. When the average diameter of the microcarrier for cell culture falls within the foregoing range, cell attachment and culture performance are excellent. Conversely, when the D50 particle diameter of the microcarrier for cell culture is less than 100 μm, the available surface area for cell culture becomes limited, potentially reducing culture efficiency; when it exceeds 300 μm, interactions among adherent cells deteriorate and the cell density in the bioreactor decreases, which may likewise lead to diminished culture efficiency.

Meanwhile, the microcarrier for cell culture may include a cell attachment-inducing layer formed on the polystyrene-based particles.

The cell attachment-inducing layer includes cell-adhesive materials that provide sites where the cell's transmembrane proteins may bind, thereby enabling adherent cells to attach, spread, and be cultured stably.

The polymer forming the cell attachment-inducing layer is not particularly limited and may include one or more polymer selected from the group consisting of gelatin, collagen, fibronectin, chitosan, poly-lysine, vitronectin, peptides containing RGD, lignin, cationic dextran, dihydroxyphenylalanine (DOPA), dopamine, norepinephrine, epinephrine, epigallocatechin, and derivatives thereof.

The microcarrier for cell culture of the embodiment may have an epoxy content in range of 40 μmol/g to 100 μmol/g.

Specifically, the microcarrier for cell culture of the embodiment may have an epoxy content of 40 μmol/g or more, 45 μmol/g or more, 49 μmol/g or more, 49.5 μmol/g or more, or 40 μmol/g or more and 100 μmol/g or less, 45 μmol/g or more and 100 μmol/g or less, 49 μmol/g or more and 100 μmol/g or less, 49.5 μmol/g or more and 100 μmol/g or less.

The epoxy content may be calculated by the following equation after adding the microcarriers for cell culture to an acidic solution, introducing an indicator solution, and titrating with NaOH.

[Equation]

$$\text{Epoxy content } (\mu\text{mol/g}) = [V_0 \text{ (ml)} - V \text{ (ml)}] \times C_{NaOH} \text{ (mol/L)}$$

wherein in this equation, $V_0$ is the volume of NaOH added to the control containing deionized water instead of particles, V is the volume of NaOH added to the actual sample, and $C_{NaOH}$ represents the concentration of NaOH used in the titration.

When the microcarriers for cell culture satisfy the above epoxy content, excellent cell adhesion may be achieved. When the epoxy content is reduced excessively, technical problems such as diminished particle dispersibility and

decreased introduction efficiency of the surface-modifying agent may arise.

Meanwhile, according to another embodiment of the disclosure, there may be provided a preparing method for microcarrier for cell culture, comprising the step of: polymerizing and recovering polystyrene-based particles from a monomer mixture including a compound represented by the following Chemical Formula 1.

In the preparing method for microcarrier for cell culture according to this embodiment, the descriptions of the polystyrene-based particles and the compound represented by Chemical Formula 1 encompass all of the aforementioned matters.

Specifically, the compound represented by Chemical Formula 1 may include at least one compound selected from the group consisting of the compounds represented by Chemical Formulas 1-1 to 1-3 below.

[Chemical Formula 1-1]

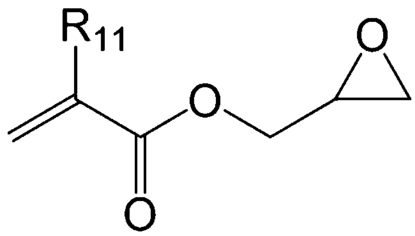

wherein in Chemical Formula 1-1, $R_{11}$ is hydrogen or an alkyl group having one or more carbon atoms,

[Chemical Formula 1-2]

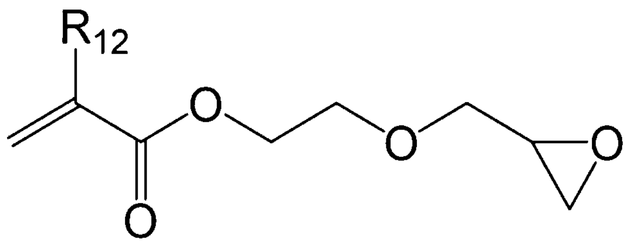

wherein in Chemical Formula 1-2, $R_{12}$ is hydrogen or an alkyl group having at least one carbon atom,

[Chemical Formula 1-3]

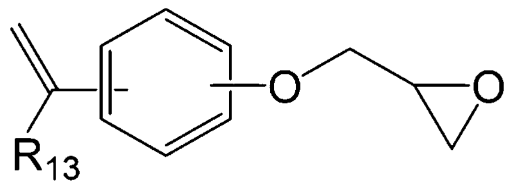

wherein in Chemical Formula 1-3, $R_{13}$ is hydrogen or an alkyl group having one or more carbon atoms.

By including at least one compound selected from the group consisting of the compounds represented by Chemical Formulas 1-1 to 1-3 as a monomer of the polystyrene-based polymer, a reaction site capable of immobilizing a cell-adhesive ligand on the surface of a microcarrier for cell culture through chemical bonding may be provided without an additional coating layer, by adjusting the weight ratio between the styrene-based monomer and the compounds represented by Chemical Formulas 1-1 to 1-3, thereby improving cell adhesiveness.

Meanwhile, the polystyrene-based particles may further include, as a monomer compound, a compound represented by the following Chemical Formula 2 in addition to the compound represented by Chemical Formula 1:

[Chemical Formula 2]

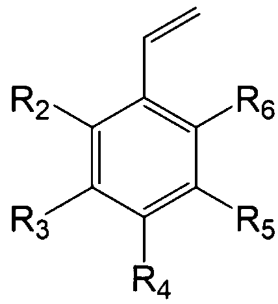

wherein in Chemical Formula 2, $R_2$ to $R_6$ are each independently hydrogen or an alkyl group having one or more carbon atoms, and at least one of $R_2$ to $R_6$ is an alkyl group having one or more carbon atoms.

By including the compound represented by the following Chemical Formula 2 as a monomer of the polystyrene-based particles, the density of the microcarrier for cell culture may be precisely controlled by adjusting the content of the compound represented by Chemical Formula 2, thereby enabling separation based on the density difference from cells, while simultaneously improving dispersibility in the cell-culture reactor or medium.

Specifically, the compound represented by Chemical Formula 2 may include at least one compound selected from the group consisting of the compounds represented by Chemical Formulas 2-1 to 2-3 below.

[Chemical Formula 2-1]

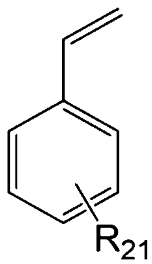

[Chemical Formula 2-2]

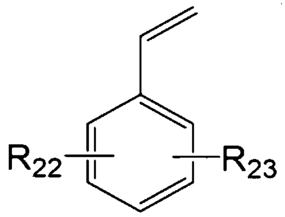

[Chemical Formula 2-3]

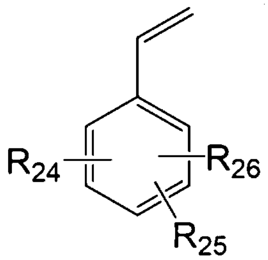

wherein in Chemical Formulas 2-1 to 2-3, $R_{21}$ to $R_{26}$ are each independently an alkyl group having one or more carbon atoms.

By including at least one compound selected from the group consisting of the compounds represented by Chemical Formulas 2-1 to 2-3 as a monomer of the polystyrene-based particles, the density of the microcarrier for cell culture may be precisely controlled by adjusting the content of the compound represented by Chemical formula 2, thereby enabling separation based on the density difference from cells and simultaneously improving dispersibility in a cell-culture reactor or medium.

More specifically, the compound represented by Chemical formula 2 may include at least one compound selected from the group consisting of the compound represented by the following Chemical Formulas 2-4 to 2-6:

[Chemical Formula 2-4]

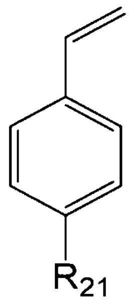

[Chemical Formula 2-5]

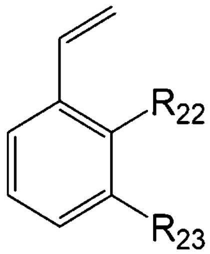

[Chemical Formula 2-6]

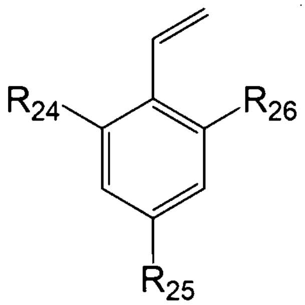

wherein in Chemical Formulas 2-4 to 2-6, $R_{21}$ to $R_{26}$ are each independently an alkyl group having one or more carbon atoms.

Meanwhile, the compound represented by Chemical Formula 2 may have a density of 0.92 g/cm$^3$ or less. Specifically, the compound represented by Chemical Formula 2 may have a density of 0.92 g/cm$^3$ or less, 0.91 g/cm$^3$ or less, 0.906 g/cm$^3$ or less, 0.9 g/cm$^3$ or less, 0.89 g/cm$^3$ or less, 0.5 g/cm$^3$ or more, 0.6 g/cm$^3$ or more, 0.7 g/cm$^3$ or more, 0.8 g/cm$^3$ or more, 0.5 g/cm$^3$ or more and 0.92 g/cm$^3$ or less, 0.6 g/cm$^3$ or more and 0.92 g/cm$^3$ or less, 0.7 g/cm$^3$ or more and 0.92 g/cm$^3$ or less, 0.8 g/cm$^3$ or more and 0.92 g/cm$^3$ or less, 0.5 g/cm$^3$ or more and 0.91 g/cm$^3$ or less, 0.6 g/cm$^3$ or more and 0.91 g/cm$^3$ or less, 0.7 g/cm$^3$ or more and 0.91 g/cm$^3$ or less, 0.8 g/cm$^3$ or more and 0.91 g/cm$^3$ or less, 0.5 g/cm$^3$ or more and 0.906 g/cm$^3$ or less, 0.6 g/cm$^3$ or more and 0.906 g/cm$^3$ or less, 0.7 g/cm$^3$ or more and 0.906 g/cm$^3$ or less, 0.8 g/cm$^3$ or more and 0.906 g/cm$^3$ or less, 0.5 g/cm$^3$ or more and 0.9 g/cm$^3$ or less, 0.6 g/cm$^3$ or more and 0.9 g/cm$^3$ or less, 0.7 g/cm$^3$ or more and 0.9 g/cm$^3$ or less, 0.8 g/cm$^3$ or more and 0.9 g/cm$^3$ or less, 0.5 g/cm$^3$ or more and 0.89 g/cm$^3$ or less, 0.6 g/cm$^3$ or more and 0.89 g/cm$^3$ or less, 0.7 g/cm$^3$ or more and 0.89 g/cm$^3$ or less, 0.8 g/cm$^3$ or more and 0.89 g/cm$^3$ or

less.

By the compound represented by Chemical Formula 2 having a density of 0.92 g/cm$^3$ or less, the density of the microcarrier for cell culture may be precisely controlled by adjusting the content of the compound represented by Chemical Formula 2, enabling separation based on the density difference from cells, while simultaneously improving dispersibility in a cell-culture bioreactor or medium.

For example, the compound represented by Chemical Formula 2 may include 4-methylstyrene, trimethylstyrene, 4-ethenyl-2-methyl-1-(2-methylpropyl)benzene, 1-ethenyl-2-methyl-4-(1-methylethyl)benzene, 1-ethenyl-2,3-dimethylbenzene, 4-(1,1-dimethylethyl)-2-ethenyl-1-methylbenzene, 2-ethenyl-4-methyl-1-(1-methylethyl)benzene, 1-ethenyl-2-(1-methylethyl)benzene, 2-tert-butylstyrene, 4-tert-butylstyrene, and 1-ethenyl-3-(1-methylethyl)benzene.

Meanwhile, the polystyrene-based particles may further include, as a monomer, a compound represented by Chemical Formula 3 in addition to the compound represented by Chemical Formula 1.

[Chemical Formula 3]

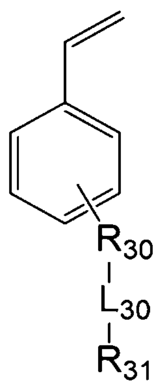

wherein in Chemical Formula 3, $L_{30}$ is -O(C=O)- or -(C=O)O-, $R_{30}$ is a direct bond or an alkylene group having one or more carbon atoms, and $R_{31}$ is an alkyl group having one or more carbon atoms.

That is, the polystyrene-based particles may include, as monomer components, the compound represented by Chemical Formula 1; or the compounds represented by Chemical Formula 1 and Chemical Formula 2; or the compounds represented by Chemical Formula 1 and Chemical Formula 3; or the compounds represented by Chemical Formula 1, Chemical Formula 2, and Chemical Formula 3.

By incorporating the compound represented by Chemical Formula 3 as a monomer of polystyrene-based particles, the substituent of the finally produced microcarrier for cell culture may be hydrolyzed, thereby improving dispersibility in a cell culture reactor or medium.

For example, the compound represented by Chemical Formula 3 may include acetoxystyrene.

Meanwhile, the monomer mixture may include the compound represented by Chemical Formula 2 in an amount of 20 parts by weight or more and 99 parts by weight or less, based on 100 parts by weight of the monomer mixture.

The monomer mixture may include both the compound represented by Chemical Formula 1 and a styrene-based monomer mixture. Specifically, based on 100 parts by weight of the monomer mixture the compound represented by Chemical Formula 2 may be included in an amount of 20 parts by weight or more, 30 parts by weight or more, 50 parts by weight or more, 75 parts by weight or more, 80 parts by weight or more, 90 parts by weight or more, 99 parts by weight or less, 95 parts by weight or less, 20 parts by weight or more and 99 parts by weight or less, 30 parts by weight or more and 99 parts by weight or less, 50 parts by weight or more and 99 parts by weight or less, 75 parts by weight or more and 99 parts by weight or less, 80 parts by weight or more and 99 parts by weight or less, 90 parts by weight or more and 99 parts by weight or less,20 parts by weight or more and 95 parts by weight or less, 30 parts by weight or more and 95 parts by weight or less, 50 parts by weight or more and 95 parts by weight or less, 75 parts by weight or more and 95 parts by weight or less, 80 parts by weight or more and 95 parts by weight or less, 90 parts by weight or more and 95 parts by weight or less.

When the compound represented by Chemical Formula 2 is included in an amount of less than 20 parts by weight based on 100 parts by weight of the monomer mixture, the particle density may increase, resulting in the loss of low-density properties. On the other hand, when it is included in an amount exceeding 99 parts by weight, the particle density may drop below 0.99 g/cm$^3$, necessitating high-speed agitation for dispersion in the medium, which may adversely affect cell characteristics.

Menwhile, the monomer mixture may include the compound represented by Chemical Formula 3 in an amount of 0.1 parts by weight or more and 5 parts by weight or less, based on 100 parts by weight of the monomer mixture.

The monomer mixture may include all of the compound represented by Chemical Formula 1, the styrene-based monomer mixture, and the ethylene-based unsaturated crosslinking agent.

Specifically, based on 100 parts by weight of the monomer mixture the monomer mixture may include the compound represented by Chemical Formula 3 in an amount of 0.1 parts by weight or more, 1 part by weight or more, 5 parts by weight or less, 4 parts by weight or less, 3 parts by weight or less, 2 parts by weight or less, 0.1 parts by weight or more and 5 parts by weight or less, 0.1 parts by weight or more and 4 parts by weight or less, 0.1 parts by weight or more and 3 parts by weight or less, 0.1 parts by weight or more and 2 parts by weight or less, 1 part by weight or more and 5 parts by weight or less, 1 parts by weight or more and 4 parts by weight or less, 1 parts by weight or more and 3 parts by weight or less, 1 parts by weight or more and 2 parts by weight or less.

When the compound represented by Chemical Formula 3 is included in an amount of less than 0.1 parts by weight based on 100 parts by weight of the monomer mixture, the surface hydrophilicity may be insufficient, leading to reduced dispersibility in the culture vessel. Conversely, when it is included in an amount exceeding 5 parts by weight, the particle density may increase, causing technical problems in which the low-density characteristics are lost.

Meanwhile, according to one embodiment of the disclosure, the step of polymerizing and recovering polystyrene-based particles from a monomer mixture including a compound represented by the following Chemical Formula 1 may include the step of polymerizing and recovering polystyrene-based particles by reacting a monomer mixture including the compound represented by Chemical Formula 1 with an ethylene-based unsaturated crosslinking agent.

The ethylene-based unsaturated crosslinking agent comprises all of the aforementioned content.

In the preparing method for microcarrier for cell culture, based on 100 parts by weight of the ethylene-based unsaturated crosslinking agent, the compound represented by Chemical Formula 2 may be included in an amount of 80 parts by weight or more and 150 parts by weight or less.

More specifically, , based on 100 parts by weight of the ethylene-based unsaturated crosslinking agent, the compound represented by Chemical Formula 2 may be included in an amount of 80 parts by weight or more, 85 parts by weight or more, 90 parts by weight or more, 150 parts by weight or less, 125 parts by weight or less, 100 parts by weight or less, 99 parts by weight or less, 80 parts by weight or more and 150 parts by weight or less, 80 parts by weight or more and 125 parts by weight or less, 80 parts by weight or more and 100 parts by weight or less, 80 parts by weight or more and 99 parts by weight or less, 85 parts by weight or more and 150 parts by weight or less, 85 parts by weight or more and 125 parts by weight or less, 85 parts by weight or more and 100 parts by weight or less, 85 parts by weight or more and 99 parts by weight or less, 90 parts by weight or more and 150 parts by weight or less, 90 parts by weight or more and 125 parts by weight or less, 90 parts by weight or more and 100 parts by weight or less, 90 parts by weight or more and 99 parts by weight or less.

By including the compound represented by Chemical Formula 2 in an amount of 80 parts by weight or more and 150 parts by weight or less base on 100 parts by weight of the ethylene-based unsaturated crosslinking agent, the low-density characteristics of the particles may be controlled due to the low density of the compound represented by the chemical formula 2, enabling separation by density difference from cells, and at the same time, dispersibility in a cell culture reactor or medium may be improved.

When the compound represented by Chemical Formula 2 is included in too small an amount relative to 100 parts by weight of the ethylene-based unsaturated crosslinker, particle density increases, causing loss of low-density characteristics and resulting in sedimentation in the medium. Conversely, when it is included in an excessively large amount, particle density falls to below 0.99 g/$cm^3$, requiring high-speed agitation to disperse the particles in the medium, which may adversely affect cell characteristics.

The monomer mixture may include the compound represented by Chemical Formula 1 and a styrene-based monomer mixture.

Based on 100 parts by weight of the styrene-based monomer mixture, the compound represented by Chemical Formula 2 may be included in an amount of 90 parts by weight or more and 100 parts by weight or less.

Specifically, based on 100 parts by weight of the styrene-based monomer mixture, the compound represented by Chemical Formula 2 may be included in an amount of 90 parts by weight or more, 91 parts by weight or more, 100 parts by weight or less, 95 parts by weight or less, or 90 parts by weight or more and 100 parts by weight or less, 91 parts by weight or more and 100 parts by weight or less, 90 parts by weight or more and 95 parts by weight or less, 91 parts by weight or more and 95 parts by weight or less.

By including the compound represented by Chemical Formula 2 in the above-mentioned amount based on 100 parts by weight of the styrene-based monomer mixture, the density of the microcarrier for cell culture may be precisely controlled, enabling separation based on the density difference from the cells while simultaneously improving dispersibility in a cell-culture bioreactor or medium.

Additionally, based on 100 parts by weight of the compound represented by Chemical Formula 2, the compound represented by Chemical Formula 1 may be included in an amount of 1 part by weight or more and 15 parts by weight or less.

Specifically, based on 100 parts by weight of the compound represented by Chemical Formula 2, the compound represented by Chemical Formula 1 may be included in an amount of 1 part by weight or more, 1.5 part by weight or more, 15 parts by weight or less, 12 parts by weight or less, 10 parts by weight or less, 6 parts by weight or less, 1 part by weight or

more and 15 parts by weight or less, 1.5 part by weight or more and 15 parts by weight or less, 1 part by weight or more and 12 parts by weight or less, 1.5 part by weight or more and 12 parts by weight or less, 1 part by weight or more and 10 parts by weight or less, 1.5 part by weight or more and 10 parts by weight or less, 1 part by weight or more and 6 parts by weight or less, 1.5 part by weight or more and 6 parts by weight or less.

When the compound represented by Chemical Formula 1 is included in an excessively small amount based on 100 parts by weight of the compound represented by Chemical Formula 2, an insufficient number of reactive sites capable of immobilizing the cell-adhesive ligand will be provided, which may lead to poor cell adhesiveness; conversely, when it is included in an excessively large amount, the density of the particles may increase, resulting in a technical problem where the low-density characteristics are lost.

Additionally, when a monomer compound represented by Chemical Formula 3 is included, the compound represented by Chemical Formula 3 may be included in an amount of 0.1 parts by weight or more and 5 parts by weight or less, based on 100 parts by weight of the ethylene-based unsaturated crosslinking agent.

More specifically, based on 100 parts by weight of the ethylene-based unsaturated crosslinking agent, the compound represented by Chemical Formula 3 may be included in an amount of 0.1 parts by weight or more, 0.5 parts by weight or more, 1 part by weight or more, 5 parts by weight or less, 3 parts by weight or less, 2 parts by weight or less, 0.1 parts by weight or more and 5 parts by weight or less, 0.5 parts by weight or more and 5 parts by weight or less, 1 part by weight or more and 5 parts by weight or less, 0.1 parts by weight or more and 3 parts by weight or less, 0.5 parts by weight or more and 3 parts by weight or less, 1 part by weight or more and 3 parts by weight or less, 0.1 parts by weight or more and 2 parts by weight or less, 0.5 parts by weight or more and 2 parts by weight or less, 1 part by weight or more and 2 parts by weight or less.

When a monomer compound represented by Chemical Formula 3 is included in an amount of 0.1 parts by weight or more and 5 parts by weight or less, based on 100 parts by weight of the ethylene-based unsaturated crosslinking agent, the dispersibility in a cell culture reactor or medium may be improved by hydrolyzing the substituents of the microcarrier for cell culture.

When the compound represented by Chemical Formula 3 is included in an amount exceeding 5 parts by weight, based on 100 parts by weight of the ethylene-based unsaturated crosslinking agent, particle density may increase, leading to a loss of low-density characteristics.

Additionally, when the monomer compound includes the compound represented by Chemical Formula 3, the compound represented by Chemical Formula 3 may be included in an amount of 0.1 parts by weight or more and 5 parts by weight or less, based on 100 parts by weight of the styrene-based monomer mixture

Specifically, based on 100 parts by weight of the styrene-based monomer mixture, the compound represented by Chemical Formula 3 may be included in an amount of 0.1 parts by weight or more, 0.5 parts by weight or more, 1 part by weight or more, 5 parts by weight or less, 3 parts by weight or less, 2.5 parts by weight or less, 0.1 parts by weight or more and 5 parts by weight or less, 0.5 parts by weight or more and 5 parts by weight or less, 1 part by weight or more and 5 parts by weight or less, 0.1 parts by weight or more and 3 parts by weight or less, 0.5 parts by weight or more and 3 parts by weight or less, 1 part by weight or more and 3 parts by weight or less, 0.1 parts by weight or more and 2.5 parts by weight or less, 0.5 parts by weight or more and 2.5 parts by weight or less, 1 part by weight or more and 2.5 parts by weight or less.

When the monomer compound includes the compound represented by Chemical Formula 3, by including the compound represented by Chemical Formula 3 in an amount of 0.1 parts by weight or more and 5 parts by weight or less based on 100 parts by weight of the styrene-based monomer mixture, the substituent of the finally produced microcarrier for cell culture may be hydrolyzed, thereby improving dispersibility in a cell culture reactor or medium.

Additionally, when the monomer compound contains a compound represented by Chemical Formula 3, the compound represented by Chemical Formula 1 may be included in an amount of 110 parts by weight or more and 500 parts by weight or less, based on 100 parts by weight of the compound represented by Chemical Formula 3.

Specifically, based on 100 parts by weight of the compound represented by Chemical Formula 3, the compound represented by Chemical Formula 1 may be included in an amount of 110 parts by weight or more, 150 parts by weight or more, 500 parts by weight or less, 400 parts by weight or less, 300 parts by weight or less, 200 parts by weight or less, 110 parts by weight or more and 500 parts by weight or less, 110 parts by weight or more and 400 parts by weight or less, 110 parts by weight or more and 300 parts by weight or less, 110 parts by weight or more and 200 parts by weight or less, 150 parts by weight or more and 500 parts by weight or less, 150 parts by weight or more and 400 parts by weight or less, 150 parts by weight or more and 300 parts by weight or less, 150 parts by weight or more and 200 parts by weight or less.

When the monomer compound contains a compound represented by Chemical Formula 3, if the compound represented by Chemical Formula 1 is included in an amount of less than 110 parts by weight based on 100 parts by weight of the compound represented by Chemical Formula 3, the compound represented by Chemical Formula 1 may be lost before polymerization due to its relatively high water solubility and may therefore not be introduced onto the particle surface. Conversely, if it is included in an amount exceeding 500 parts by weight, the particle density may become higher, thereby losing low-density characteristics.

In the preparing method for microcarrier for cell culture of the embodiment, the step of polymerizing and

recovering polystyrene-based particles from a monomer mixture including a compound represented by the Chemical Formula 1 may include the step of performing a suspension polymerization reaction of the monomer composition and recovering the product of the suspension polymerization reaction.

More specifically, the performing a suspension polymerization reaction of the monomer composition may include: a step of mixing the monomer composition with an aqueous dispersion, applying shear force to homogenize the monomer composition into droplets in the aqueous dispersion; and a step of carrying out suspension polymerization of the homogenized monomer composition at a stirring speed of 300 rpm to 1000 rpm.

During the step of homogenizing the monomer composition into droplets in the aqueous dispersion, stirring may be performed at a speed of 300 rpm to 1000 rpm, or 400 rpm to 800 rpm.

In the step of carrying out a suspension polymerization of the homogenized monomer composition at a stirring speed of 300 rpm to 1000 rpm, or 400 rpm to 800 rpm, the particle structure of the polystyrene may further reduce the density of the microcarrier owing to the structure of the compound represented by Chemical Formula 1, while still enabling the production of microcarriers with a high proportion of intact, perfectly spherical particles without damage or breakage.

In the step of carrying out a suspension polymerization of the homogenized monomer composition at a stirring speed of 300 rpm to 1000 rpm, or 400 rpm to 800 rpm, the suspension polymerization conditions are not particularly limited; for example, the process may be conducted in a range of 50 °C to 100 °C for 3 hours to 18 hours.

Meanwhile, the preparing method for microcarrier for cell culture may further comprise the step of washing and drying steps after the step of polymerizing and recovering polystyrene-based particles from a monomer mixture containing the compound represented by Chemical Formula 1.

Specifically, the washing step may include filtering the reaction product through a sieve of 30 μm to 100 μm and subsequently stirring it in 100 % ethanol at room temperature five to seven times.

The drying step may include placing the washed product in a vacuum oven and vacuum-drying at room temperature; however, this is not limiting, and any conventionally known drying method may be employed without particular restriction.

In one embodiment, a preparing method for microcarriers for cell culture includes the step of introducing a cell attachment-inducing layer onto polystyrene-based particles.

Meanwhile, in the present disclosure, a cell-adhesive material may be introduced onto the particle surface through chemical bonding. For example, the method may include modifying the particles with a solution containing one or more compound selected from the group consisting of gelatin, collagen, fibronectin, chitosan, poly-L-lysine, vitronectin, peptides including RGD, lignin, cationic dextran, dihydroxyphenylalanine (DOPA), dopamine, norepinephrine, epinephrine, epigallocatechin, and derivatives thereof.

A solution containing one or more compound selected from the group consisting of gelatin, collagen, fibronectin, chitosan, poly-L-lysine, vitronectin, peptides including RGD, lignin, cationic dextran, dihydroxyphenylalanine (DOPA), dopamine, norepinephrine, epinephrine, epigallocatechin, and derivatives thereof may act as an adhesion factor that bonds cells to the microcarrier, thereby enhancing the attachment between the cells and the microcarrier and making it more suitable for large-scale cell culture.

Specifically, the step of introducing the cell-attachment-inducing layer may comprise immersing the surface of the polystyrene-based particles in a solution containing one or more compound selected from the group consisting of gelatin, collagen, fibronectin, chitosan, poly-L-lysine, vitronectin, peptides containing RGD, lignin, cationic dextran, dihydroxyphenylalanine (DOPA), dopamine, norepinephrine, epinephrine, epigallocatechin, and derivatives thereof, and allowing them to react for 10 to 20 hours, or 15 to 20 hours, or 17 to 19 hours.

The microcarrier for cell culture prepared by the above preparing method may have an epoxy content in range of 40 μmol/g to 100 μmol/g.

Specifically, the microcarrier for cell culture of the embodiment may have an epoxy content of 40 μmol/g or more, 45 μmol/g or more, 49 μmol/g or more, 49.5 μmol/g or more, or 40 μmol/g or more and 100 μmol/g or less, 45 μmol/g or more and 100 μmol/g or less, 49 μmol/g or more and 100 μmol/g or less, 49.5 μmol/g or more and 100 μmol/g or less.

The epoxy content may be calculated by the following equation after adding the microcarriers for cell culture to an acidic solution, introducing an indicator solution, and titrating with NaOH.

[Equation]

$$\text{Epoxy content } (\mu\text{mol/g}) = [V_0 \text{ (ml)} - V \text{ (ml)}] \times C_{NaOH} \text{ (mol/L)}$$

wherein in this equation, $V_0$ is the volume of NaOH added to the control containing deionized water instead of particles, V is the volume of NaOH added to the actual sample, and $C_{NaOH}$ represents the concentration of NaOH used in the titration.

When the microcarriers for cell culture satisfy the above epoxy content, excellent cell adhesion may be achieved. When the epoxy content is reduced excessively, technical problems such as diminished particle dispersibility and decreased introduction efficiency of the surface-modifying agent may arise.

According to another embodiment of the disclosure a cell culture composition comprising cells and the microcarrier for cell culture of the aforementioned embodiment may be provided. All details described in the above embodiment regarding the microcarrier for cell culture are incorporated herein.

The cells are adherent animal cells, without particular limitation thereto, and may include, for example, at least one cell selected from the group consisting of the fibroblasts, epithelial cells, osteoblasts, chondrocytes, hepatocytes, human umbilical cord blood-derived cells, human bone marrow-derived mesenchymal stem cells, CHO (Chinese hamster ovary) cells, kidney cells (HEK293, BHK21, MDCK, Vero cells, etc.), or a mixture of two or more thereof.

In addition, the apparent density difference between the microcarrier for cell culture and the cells may be 0.20 g/$cm^3$ or less. Satisfying a density difference of 0.20 g/$cm^3$ or less allows the cells and the microcarrier to be easily separated after culture by exploiting the difference in sedimentation velocity under gravity when they are recovered, while also preventing the problem in which, at the early stage of culture, the microcarrier floats only on the surface of the culture medium, making cell attachment difficult.

The cell-culture composition may further include a medium solution. This medium solution may include various additives that sufficiently satisfy nutrients and environmental conditions such as pH, temperature, and osmotic pressure, closely mimicking physiological conditions based on body fluids such as plasma or lymph. Any of a wide range of substances well known in the field of cell-culture technology may be used without restriction.

For example, in one embodiment, the microcarrier for cell culture has a lower density than the medium solution, so when it is introduced into the medium solution under agitation, it remains suspended within the solution. As the number of cells adhering to the surface of the low-density microcarrier increases, the density of the cell-laden microcarrier (hereinafter referred to as the "microcarrier-cell conjugate") gradually rises, causing it to settle within the medium solution.

Therefore, after treating the cell-laden microcarrier (microcarrier-cell conjugate) with a cell-detachment enzyme, it may be separated by centrifugation. By detaching the cells from the microcarrier-cell conjugate, the cultured cells may be readily collected.

**[Advantageous Effects]**

According to the present disclosure, a microcarrier for cell culture having high surface hydrophilicity and enhanced cell-attachment properties, improved dispersibility in a cell-culture reactor or medium, a preparing method for the microcarrier, and a cell-culture method using the same may be provided.

**[BRIEF DESCRIPTION OF THE DRAWINGS]**

Fig. 1 is an SEM image of the surface morphology of the microcarrier for cell culture according to Example 1.

**[DETAILED DESCRIPTION OF THE EMBODIMENTS]**

Hereinafter, embodiments of the present disclosure will be described in more detail with reference to the following Examples. However, the following Examples are for illustrative purposes only, and are not intended to limit the scope of the present disclosure.

**Example 1**

Polyvinyl alcohol (molecular weight 85-124 K, 87-89 % hydrolysis) was dissolved in distilled water at a concentration of 2 wt% to prepare an aqueous dispersion, which was stirred at room temperature for 20 minutes.

Styrene, glycidyl methacrylate, and t-butylstyrene as monomers and divinylbenzene as cross-linker were mixed at a weight ratio of 0.05:0.05:0.9:1 and fully dissolved; 25 g of this mixture was then supplemented with 2 wt% of the initiator V-65 (based on the total weight of monomers and cross-linker) and stirred for an additional 5 minutes to obtain the monomer composition.

A 1 L reactor was charged with 600 g of the aqueous dispersion, to which the monomer composition was added. Shear was applied to the aqueous dispersion and the monomer composition at 400 rpm at room temperature, dispersing the monomer composition into the aqueous phase as fine droplets and thereby homogenizing the mixture.

The homogenized mixture was stirred at 400 rpm and reacted under nitrogen purging at 85 °C for 6 h to produce polystyrene particles. The particles were washed twice with 60 °C distilled water and five times with ethanol, then recovered by drying in an 80 °C oven. The recovered polystyrene particles were used as microcarriers for cell culture.

The physical properties of the polystyrene particles are as follows.

Average diameter: 178 μm (D50 measured using PSA equipment)
Apparent density: 0.99-1.04 g/$cm^3$

**Example 2**

A microcarrier for cell culture was prepared in the same manner as in Example 1, except that the weight ratio of styrene, glycidyl methacrylate, t-butyl styrene as monomers and divinylbenzene as cross-linker was adjusted to 0.07 : 0.03 : 0.9 : 1

**Example 3**

A microcarrier for cell culture was prepared in the same manner as in Example 1, except that the weight ratio of styrene, glycidyl methacrylate, t-butyl styrene as monomers and divinylbenzene as cross-linker was adjusted to 0.085:0.015:0.9:1.

**Example 4**

A microcarrier for cell culture was prepared in the same manner as in Example 1, except that the weight ratio of styrene, glycidyl methacrylate, t-butyl styrene, acetoxystyrene (density: 1.06 g/$cm^3$) as monomers and divinylbenzene as cross-linker was adjusted to 0.06:0.02:0.91:0.01:1.

**Example 5**

A microcarrier for cell culture was prepared in the same manner as in Example 1, except that the weight ratio of styrene, glycidyl methacrylate, t-butyl styrene, acetoxystyrene (density: 1.06 g/$cm^3$) as monomers and divinylbenzene as cross-linker was adjusted to 0.05:0.03:0.90:0.02:1.

**Example 6**

A microcarrier for cell culture was prepared in the same manner as in Example 1, except that 2-(2-oxiranylmethoxy)ethyl methacrylate (density: 1.1 g/$cm^3$) was used in place of the monomer glycidyl methacrylate.

**Example 7**

A microcarrier for cell culture was prepared in the same manner as in Example 1, except that omitting styrene as a monomer, replacing glycidyl methacrylate with 4-vinylphenylglycidyl ether, and adjusting the weight ratio of t-butylstyrene, 4-vinylphenylglycidyl ether (density: 1.1 g/$cm^3$ as monomers and divinylbenzene as cross-linker to 0.9 : 0.1 : 1.

**Example 8**

A microcarrier for cell culture was prepared in the same manner as in Example 1, except that the weight ratio of styrene, glycidyl methacrylate, t-butyl styrene as monomers and divinylbenzene as cross-linker was adjusted to 0.1:0.1:0.8:1.

**Example 9**

A microcarrier for cell culture was prepared in the same manner as in Example 1, except that the weight ratio of styrene, glycidyl methacrylate, t-butyl styrene as monomers and divinylbenzene as cross-linker was adjusted to 0.15:0.05:0.8:1.

**Example 10**

A microcarrier for cell culture was prepared in the same manner as in Example 1, except that the weight ratio of styrene, glycidyl methacrylate, t-butyl styrene as monomers and divinylbenzene as cross-linker was adjusted to 0.075:0.075:0.85:1.

**Example 11**

A microcarrier for cell culture was prepared in the same manner as in Example 1, except that the weight ratio of styrene, glycidyl methacrylate, t-butyl styrene as monomers and divinylbenzene as cross-linker was adjusted to 0.1:0.05:0.85:1.

**Example 12**

A microcarrier for cell culture was prepared in the same manner as in Example 1, except that the weight ratio of styrene, glycidyl methacrylate, t-butyl styrene as monomers and divinylbenzene as cross-linker was adjusted to 0.02:0.08:0.9:1.

**Example 13**

A microcarrier for cell culture was prepared in the same manner as in Example 1, except that the weight ratio of styrene, glycidyl methacrylate, t-butyl styrene as monomers and divinylbenzene as cross-linker was adjusted to 0.9:0.1:0:1.

**Comparative Example 1**

A microcarrier for cell culture was prepared in the same manner as in Example 1, except that the weight ratio of styrene, glycidyl methacrylate, t-butyl styrene as monomers and divinylbenzene as cross-linker was adjusted to 0.1:0:0.9:1.

**Experimental Example: Measurement** of **Physical Properties** of **Microcarriers** for Cell Culture

For the microcarriers for cell culture obtained in the Examples and Comparative Example, their physical properties were measured by the following methods, and the results are shown in Tables 1 and 2.

Experiment 1. Average Particle Size (unit: $\mu$m)

For the microcarriers for cell culture obtained in the Examples and Comparative Example, the particles were dispersed in ethanol at a 10 wt% level, after which the D50 particle diameter (the particle size corresponding to the cumulative distribution percentage reaching 50%) was measured using a PSA (Particle Size Analysis) instrument.

Experiment 2. Epoxy Content Analysis

A 0.1 g of the microcarrier for cell culture prepared in the Examples and Comparative Example was dispersed in an HCl/acetone solution (volume ratio = 1:80) and sonicated for 4 minutes.
After adding two drops of an indicator solution (0.1 wt % cresol red + 0.1 wt % thymol blue, volume ratio 1:3, pH 7, 0.01 M NaOH), titration was carried out with 0.1 M NaOH.
The epoxy content (µmol/g) was calculated using the following equation.

[Equation]

$$\text{Epoxy content } (\mu\text{mol/g}) = [V_0 \text{ (ml)} - V \text{ (ml)}] \times C_{NaOH} \text{ (mol/L)}$$

Wherein in this equation, $V_0$ is the volume of NaOH added to the control containing 0.1 g of DIW instead of the particles, V is the volume of NaOH added to the actual sample, and $C_{NaOH}$ is the concentration of the NaOH used for the titration.

**Experiment 3. Dispersibility of particles in the bioreactor**

1g of the microcarrier for cell culture prepared in the Examples and Comparative Example was dispersed in 4 mL of 1× PBS, after which 10 mg of the GRGDSK peptide was added and allowed to react for 18 hours. The peptide-coated microcarriers were then washed three times with 1× PBS.
Prior to culture, the required amount of these microcarriers was pre-dispersed with medium in a 20 mL glass vial and wetted for approximately 10-18 hours. The pre-dispersed microcarriers were filtered through a cell strainer, introduced into a 100 mL 3D bioreactor together with 60 mL of medium, and a culture medium containing mesenchymal stem cells (density: 1.05 g/$cm^3$) was added, followed by incubation for 24 hours. (Culture conditions: 37 °C, 5 % $CO_2$ incubator, bioreactor stirred at 25 rpm)
After 24 hours of incubation, particles that remained undispersed and floated on the medium's surface in the incubator were removed by filtration. The particles dispersed within the incubator were thoroughly washed with DPBS or water, dried, weighed, and the degree of dispersion was calculated as the percentage of the dispersed particle weight

relative to the total weight. Evaluation was carried out according to the following criteria.

Excellent: Dispersibility of 90 % or more and up to 100 %
Good: Dispersibility of 80 % or more and up to 90 %
Moderate: Dispersibility of 60 % or more and less than 80 %
Poor: Dispersibility of less than 60 %

**Experiment 4. Initial Cell Attachment**

1g of the microcarrier for cell culture prepared in the Examples and Comparative Example was dispersed in 4 mL of 1× PBS, after which 10 mg of GRGDSK peptide was added and allowed to react for 18 hours. The peptide-coated microcarrier was then obtained by washing three times with 1× PBS.

Prior to culture, 1 g of the microcarrier required for the experiment was pre-dispersed with medium in a 20 mL glass vial and wetted for approximately 10-18 hours. The pre-dispersed microcarrier was passed through a cell strainer, combined with 60 mL of medium, and transferred to a 100 mL 3D bioreactor. Culture medium containing 1,800,000 mesenchymal stem cells (density: 1.05 g/cm$^3$) was added, and the cells were cultured for 24 hours under the following conditions: 37 °C, 5 % $CO_2$ incubator, and bioreactor agitation at 25 rpm.

After 24 hours of cultivation, 1 mL of the particle suspension in the bioreactor vessel was collected. Using a Nucleocounter NC-200 (Chemometec), the percentage of cells attached to the microcarriers relative to the total cell count was determined and assessed according to the following criteria:

Excellent: 93 %-100 % of the total cells attached to the microcarriers
Good: ≥ 85 % to < 93 % of the total cells attached to the microcarriers
Moderate: ≥ 60 % to < 85 % of the total cells attached to the microcarriers
Poor: < 60 % of the total cells attached to the microcarriers

[Table 1]

| Category | Average Particle Size (μm, D50) | Epoxy Content (μmol/g) | Dispersibility (%) | Initial Cell Attachment (%) |
|---|---|---|---|---|
| Example 1 | 178 | 97.8 | Excellent | Excellent |
| Example 2 | 155 | 75.1 | Excellent | Excellent |
| Example3 | 162 | 49.7 | Good | Good |
| Example4 | 184 | 51.1 | Good | Good |
| Example5 | 189 | 72.9 | Good | Good |
| Example6 | 190 | 77.5 | Excellent | Good |
| Example7 | 210 | 49.4 | Excellent | Excellent |
| Example8 | 172 | 49.3 | Good | Moderate |
| Example9 | 180 | 19.3 | Good | Moderate |
| Example10 | 168 | 99.3 | Good | Moderate |
| Example11 | 177 | 79.7 | Good | Moderate |
| Example12 | 172 | 59.8 | Good | Good |
| Example13 | 182 | 170.1 | Good | Moderate |
| Comparative Example 1 | 163 | 0 | Poor | Poor |

As shown in Table 1, the microcarrier for cell culture of the embodiment exhibited excellent cell adhesion due to improved particle dispersibility under culture conditions and its ability to react with ligand peptides for cell attachment.

In Comparative Example 1, both dispersibility and initial cell attachment were found to be inferior to those of the embodiment.

**Experiment 5. Apparent density (unit: g/cm$^3$)**

The microcarriers for cell culture manufactured in the above Examples and Comparative Examples were added to either distilled water (density: 0.99 g/cm$^3$) or a cell culture medium (density: 1.01 g/cm$^3$) at room temperature (25 °C) and atmospheric pressure (1 atm). The apparent density was evaluated by observing whether the particles floated or settled. The cell culture medium was prepared with 94.9 wt% Advanced MEM, 5 wt% Fetal Bovine Serum, and 1 wt% Gentamicin.
For the microcarrier for cell culture prepared in the Examples and Comparative Example, the apparent density was evaluated at room temperature (25 °C) and atmospheric pressure (1 atm) by adding the particles to ethanol aqueous solutions with densities of 0.985 g/cm$^3$, 0.99 g/cm$^3$, and 0.997 g/cm$^3$ and to glycerol aqueous solutions with densities of 1.02 g/cm$^3$ and 1.04 g/cm$^3$, and observing whether they floated or sank according to the criteria below.

1) 0.985 g/cm$^3$ < d < 0.99 g/cm$^3$ (greater than 0.985 g/cm$^3$ and less than 0.99 g/cm$^3$)
Sinks in an ethanol aqueous solution with a density of 0.985 g/cm$^3$ and floats in an ethanol aqueous solution with a density of 0.99 g/cm$^3$.
2) 0.99 g/cm$^3$ < d < 1.02 g/cm$^3$ (greater than 0.99 g/cm$^3$ and less than 1.02 g/cm$^3$)
Sinks in an ethanol aqueous solution with a density of 0.99 g/cm$^3$ and floats in a glycerol aqueous solution with a density of 1.02 g/cm$^3$.
3) 0.99 g/cm$^3$ < d < 1.04 g/cm$^3$ (greater than 0.99 g/cm$^3$ and less than 1.04 g/cm$^3$)
Sinks in an ethanol aqueous solution with a density of 0.99 g/cm$^3$ and floats in a glycerol aqueous solution with a density of 1.04 g/cm$^3$.
4) 0.99 g/cm$^3$ < d < 1.01 g/cm$^3$ (greater than 0.99 g/cm$^3$ and less than 1.01 g/cm$^3$)
Sinks in distilled water with a density of 0.99 g/cm$^3$ and floats in a cell culture medium with a density of 1.01 g/cm$^3$.
5) d > 1.04 g/cm$^3$ (greater than 1.04 g/cm$^3$)
Sinks in a glycerol aqueous solution with a density of 1.04 g/cm$^3$.

**Experiment 6. Possibility of separation by density difference**

For the microcarrier for cell culture prepared in the Examples and Comparative Example, the possibility of separating the cells from the microcarriers for cell culture by means of density difference was evaluated under ambient temperature (25 °C) and atmospheric pressure (1 atm) by centrifuging a mixture of the cell culture medium and the microcarriers as follows.

O: No particles sank at the bottom after centrifugation.
X: Particles sank at the bottom after centrifugation.

**Experiment** 7. **Observation of Surface Morphology**

The surface morphology of the microcarrier for cell culture prepared in the Example 1 was examined using a scanning electron microscope (SEM, JSM-7610F, JEOL), and the results are shown in Figure 1.

[Table 2]

| Category | Apparent density (g/cm$^3$) | Possibility of separation by density difference |
|---|---|---|
| Example 1 | 0.99~1.04 | O |
| Example 2 | 0.99~1.04 | O |
| Example3 | 0.99~1.04 | O |
| Example4 | 0.99~1.04 | O |
| Example5 | 0.99~1.04 | O |
| Example6 | 0.99~1.04 | O |
| Example7 | 0.99 - 1.04 | O |
| Example8 | Over 1.04 | X |
| Example9 | Over 1.04 | X |
| Example10 | Over 1.04 | X |
| Example11 | Over 1.04 | X |

(continued)

| Category | Apparent density (g/cm$^3$) | Possibility of separation by density difference |
|---|---|---|
| Example12 | Over 1.04 | X |
| Example 13 | Over 1.04 | X |
| Comparative Example 1 | less than 0.99 | O |

As shown in Table 2, the microcarriers for cell culture in Examples 1-7, with densities of 1.04 g/cm$^3$ or less, are suitable for cell culture and may be separated under culture conditions based on density differences. In contrast, the particles in Examples 8-13 have densities exceeding that of the culture medium and therefore lack low-density characteristics, making separation by density impossible. In Comparative Example 1, the apparent density was excessively low, so the microcarriers floated only on the surface of the culture medium during the initial culture stage, potentially hindering cell attachment.

**Claims**

1. A microcarrier for cell culture, comprising polystyrene-based particles that include a monomer compound represented by Chemical Formula 1:

[Chemical Formula 1]

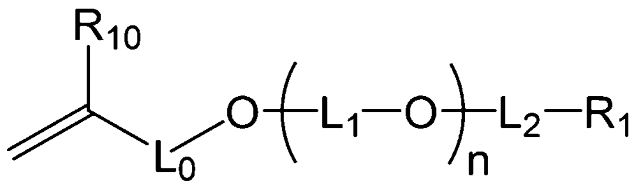

wherein in Chemical Formula 1,
$L_0$ is an arylene group having 6 or more carbon atoms or -(C=O)-,
$L_1$ and $L_2$ are each independently an alkylene group having 1 or more carbon atoms,
$R_1$ is a reactive functional group capable of ring-opening reaction,
$R_{10}$ is hydrogen or an alkyl group having 1 or more carbon atoms, and
n is an integer of 0 or greater.

2. The microcarrier for cell culture of claim 1, wherein:

the polystyrene-based particle comprises a reaction product of the compound represented by Chemical Formula 1 and an ethylene-based unsaturated crosslinking agent, and
includes 1 part by weight or more and 15 parts by weight or less of the compound represented by Chemical Formula 1, based on 100 parts by weight of the ethylene-based unsaturated crosslinking agent.

3. The microcarrier for cell culture of claim 1, wherein:

the compound represented by Chemical Formula 1 includes at least one compound selected from the group consisting of the compounds represented by Chemical Formulas 1-1 to 1-3:

[Chemical Formula 1-1]

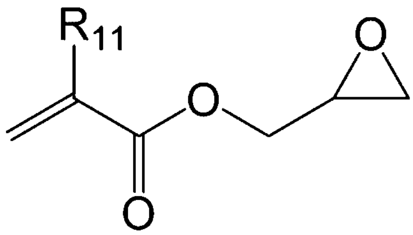

wherein in Chemical Formula 1-1,
$R_{11}$ is hydrogen or an alkyl group having one or more carbon atoms,

[Chemical Formula 1-2]

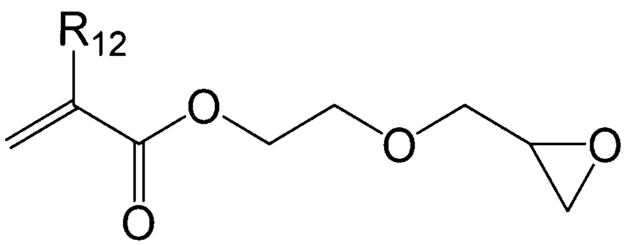

wherein in Chemical Formula 1-2,
$R_{12}$ is hydrogen or an alkyl group having at least one carbon atom,

[Chemical Formula 1-3]

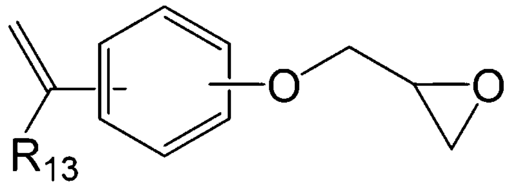

wherein in Chemical Formula 1-3,
$R_{13}$ is hydrogen or an alkyl group having one or more carbon atoms.

**4.** The microcarrier for cell culture of claim 1, wherein:

the polystyrene-based particles include a compound represented by the following Chemical Formula 2 as a monomer:

[Chemical Formula 2]

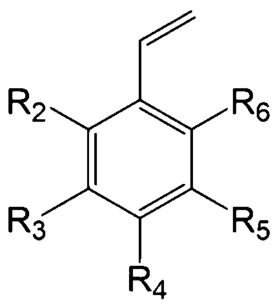

wherein in Chemical Formula 2,

$R_2$ to $R_6$ are each independently hydrogen or an alkyl group having one or more carbon atoms, and at least one of $R_2$ to $R_6$ is an alkyl group having one or more carbon atoms.

**5.** The microcarrier for cell culture of claim 4, wherein:

the compound represented by Chemical Formula 2 includes at least one compound selected from the group consisting of the compounds represented by Chemical Formulas 2-1 to 2-3:

[Chemical Formula 2-1]

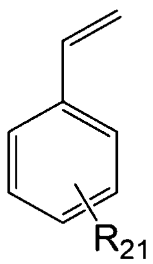

[Chemical Formula 2-2]

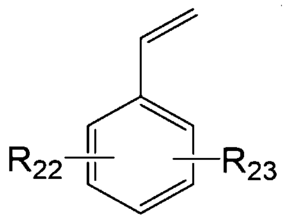

[Chemical Formula 2-3]

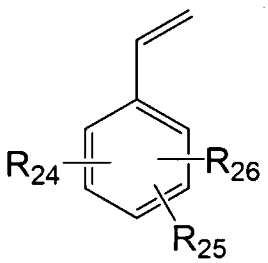

wherein in Chemical Formulas 2-1 to 2-3,
$R_{21}$ to $R_{26}$ are each independently an alkyl group having one or more carbon atoms.

**6.** The microcarrier for cell culture of claim 4, wherein:
the compound represented by Chemical Formula 2 has a density of 0.92 $g/cm^3$ or less.

**7.** The microcarrier for cell culture of claim 4, wherein:

the polystyrene-based particles comprise the reaction product of the compound represented by Chemical Formula 1, a styrene-based monomer mixture, and an ethylene-based unsaturated crosslinking agent, and includes the compound represented by Chemical Formula 2 in an amount of 80 parts by weight or more and 150 parts by weight or less based on 100 parts by weight of the ethylene-based unsaturated crosslinking agent.

**8.** The microcarrier for cell culture of claim 4, wherein:

the polystyrene-based particles comprise the reaction product of the compound represented by Chemical

Formula 1, a styrene-based monomer mixture, and an ethylene-based unsaturated crosslinking agent, and includes the compound represented by Chemical Formula 2 in an amount of 90 parts by weight or more and 100 parts by weight or less based on 100 parts by weight of the styrene-based monomer mixture.

**9.** The microcarrier for cell culture of claim 4, wherein:

the polystyrene-based particles comprise the reaction product of the compound represented by Chemical Formula 1, a styrene-based monomer mixture, and an ethylene-based unsaturated crosslinking agent, and includes the compound represented by Chemical Formula 1 in an amount of 1 parts by weight or more and 15 parts by weight or less based on 100 parts by weight of the compound represented by Chemical Formula 2.

**10.** The microcarrier for cell culture of Claim 1, wherein:

the polystyrene-based particles include a compound represented by the following Chemical Formula 3 as a monomer:

[Chemical Formula 3]

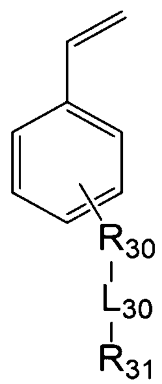

wherein in Chemical Formula 3,
$L_{30}$ is -O(C=O)- or -(C=O)O-,
$R_{30}$ is a direct bond or an alkylene group having one or more carbon atoms, and
$R_{31}$ is an alkyl group having one or more carbon atoms.

**11.** The microcarrier for cell culture of Claim 10, wherein:

the polystyrene-based particles comprise the reaction product of the compound represented by Chemical Formula 1, a styrene-based monomer mixture, and an ethylene-based unsaturated crosslinking agent, and includes the compound represented by Chemical Formula 3 in an amount of 0.1 parts by weight or more and 5 parts by weight or less based on 100 parts by weight of the ethylene-based unsaturated crosslinking agent.

**12.** The microcarrier for cell culture of Claim 10, wherein:

the polystyrene-based particles comprise the reaction product of the compound represented by Chemical Formula 1, a styrene-based monomer mixture, and an ethylene-based unsaturated crosslinking agent, and includes the compound represented by Chemical Formula 3 in an amount of 0.1 parts by weight or more and 5 parts by weight or less based on 100 parts by weight of the styrene-based monomer mixture.

**13.** The microcarrier for cell culture of Claim 10, wherein:

the polystyrene-based particles comprise the reaction product of the compound represented by Chemical Formula 1, a styrene-based monomer mixture, and an ethylene-based unsaturated crosslinking agent, and includes the compound represented by Chemical Formula 1 in an amount of 110 parts by weight or more and 500 parts by weight or less based on 100 parts by weight of the compound represented by Chemical Formula 3.

**14.** The microcarrier for cell culture of Claim 1, wherein:

comprises a cell attachment-inducing layer formed on the polystyrene-based particles.

**15.** The microcarrier for cell culture of Claim 1, wherein:
the apparent density of the microcarrier for cell culture is in a range of 0.99 $g/cm^3$ to 1.04 $g/cm^3$.

**16.** The microcarrier for cell culture of Claim 1, wherein:
the D50 particle diameter of the cell culture microcarrier is in a range of 100 $\mu m$ to 300 $\mu m$.

**17.** A preparing method for microcarrier for cell culture,

comprising the step of polymerizing and recovering polystyrene-based particles from a monomer mixture including a compound represented by the following Chemical Formula 1:

[Chemical Formula 1]

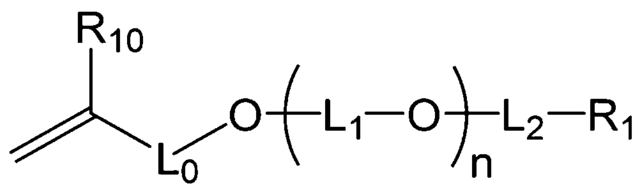

wherein in Chemical Formula 1,
$L_0$ is an arylene group having 6 or more carbon atoms or -(C=O)-,
$L_1$ and $L_2$ are each independently an alkylene group having 1 or more carbon atoms,
$R_1$ is a reactive functional group capable of ring-opening reaction,
$R_{10}$ is hydrogen or an alkyl group having 1 or more carbon atoms, and
n is an integer of 0 or greater.

**18.** The preparing method for microcarrier for cell culture of Claim 17, wherein:

the monomer mixture comprises a compound represented by the following Chemical Formula 2:

[Chemical Formula 2]

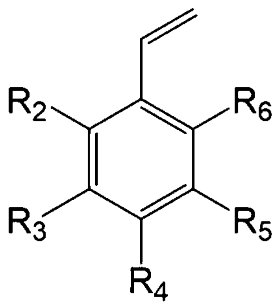

wherein in Chemical Formula 2,
$R_2$ to $R_6$ are each independently hydrogen or an alkyl group having one or more carbon atoms, and at least one of $R_2$ to $R_6$ is an alkyl group having one or more carbon atoms.

**19.** The preparing method for microcarrier for cell culture of Claim 17, wherein:

the monomer mixture comprises a compound represented by the following Chemical Formula 3:

[Chemical Formula 3]

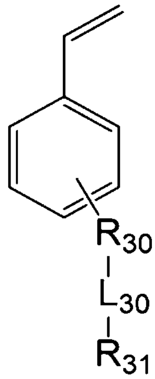

wherein in Chemical Formula 3,
$L_{30}$ is -O(C=O)- or -(C=O)O-,
$R_{30}$ is a direct bond or an alkylene group having one or more carbon atoms, and
$R_{31}$ is an alkyl group having one or more carbon atoms.

**20.** The preparing method for microcarrier for cell culture of Claim 17, wherein:
comprises the step of introducing the cell-adhesion-inducing layer onto the polystyrene-based particle.

**21.** A cell culture composition comprising cells and the microcarrier for cell culture of claim 1.

**22.** The cell culture composition of Claim 21, wherein:
the cell comprises at least one compound selected from the group consisting of the fibroblasts, epithelial cells, osteoblasts, chondrocytes, hepatocytes, human umbilical cord blood-derived cells, human bone marrow-derived mesenchymal stem cells, CHO cells, kidney cells.

**23.** The cell culture composition of Claim 21, wherein:
the difference in apparent density between the microcarrier for cell culture and the cells is 0.20 g/cm$^3$ or less.

FIG. 1

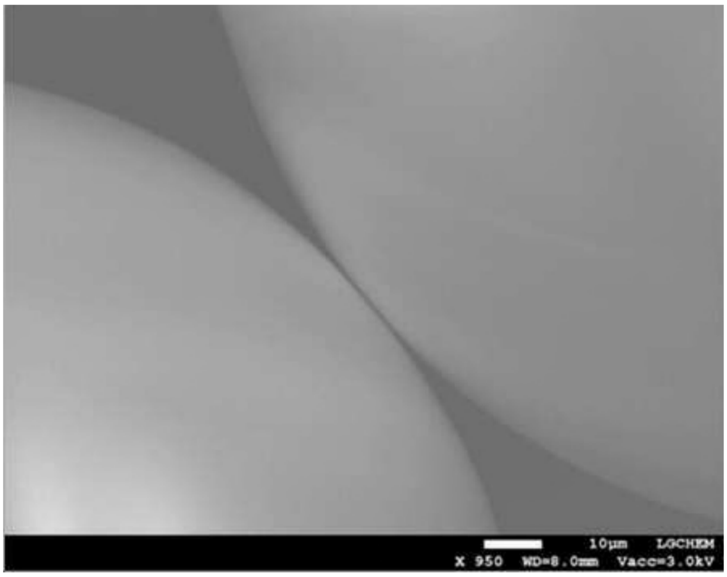

**INTERNATIONAL SEARCH REPORT**

International application No. **PCT/KR2025/095362**

**A. CLASSIFICATION OF SUBJECT MATTER**

**C12N 5/00**(2006.01)i; **C12M 1/12**(2006.01)i

According to International Patent Classification (IPC) or to both national classification and IPC

**B. FIELDS SEARCHED**

Minimum documentation searched (classification system followed by classification symbols)

C12N 5/00(2006.01); C08F 212/08(2006.01); C08F 212/12(2006.01); C12N 5/06(2006.01); C12N 5/0775(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 세포 배양(cell culture), 마이크로캐리어(microcarrier), 폴리스티렌 입자(polystyrene particle), 밀도(density), 글리시딜 메타크릴레이트(glycidyl methacrylate), t-부틸스티렌(t-butylstyrene), 아세톡시스티렌(acetoxystyrene), 디비닐벤젠(divinylbenzene)

**C. DOCUMENTS CONSIDERED TO BE RELEVANT**

| Category* | Citation of document, with indication, where appropriate, of the relevant passages | Relevant to claim No. |
|---|---|---|
| X | US 5902834 A (PORRVIK, I.) 11 May 1999 (1999-05-11)<br>column 1, lines 10-16; column 2, lines 50-54; column 5, lines 16-67 | 1-3,14-17,20-23 |
| Y | | 4-13,18,19 |
| Y | US 6214618 B1 (HILLEGAS, W. J. et al.) 10 April 2001 (2001-04-10)<br>claims 1, 2, 9, 10; column 5, lines 25-27 | 4-9,18 |
| Y | WO 2007-125288 A1 (UNIVERSITY OF DURHAM et al.) 08 November 2007 (2007-11-08)<br>claims 1, 10-12, 19, 20 | 10-13,19 |
| A | CN 102516437 A (NINGBO INSTITUTE OF TECHNOLOGY, ZHEJIANG UNIVERSITY) 27 June 2012 (2012-06-27)<br>claims 1-4 | 1-23 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 August 2025** | **27 August 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2025/095362**

**C. DOCUMENTS CONSIDERED TO BE RELEVANT**

| Category* | Citation of document, with indication, where appropriate, of the relevant passages | Relevant to claim No. |
|---|---|---|
| A | KR 10-2023-0049009 A (KOREA ADVANCED INSTITUTE OF SCIENCE AND TECHNOLOGY) 12 April 2023 (2023-04-12)<br>entire document | 1-23 |

Form PCT/ISA/210 (second sheet) (July 2022)

**INTERNATIONAL SEARCH REPORT**
**Information on patent family members**

International application No.
**PCT/KR2025/095362**

| Patent document cited in search report | Publication date (day/month/year) | Patent family member(s) | Publication date (day/month/year) |
|---|---|---|---|
| US 5902834 A | 11 May 1999 | EP 0763064 A1 | 19 March 1997 |
| | | EP 0763064 B1 | 01 August 2001 |
| | | JP 10-500164 A | 06 January 1998 |
| | | JP 3608625 B2 | 12 January 2005 |
| | | WO 95-31485 A1 | 23 November 1995 |
| US 6214618 B1 | 10 April 2001 | None | |
| WO 2007-125288 A1 | 08 November 2007 | AU 2007-245453 A1 | 08 November 2007 |
| | | AU 2007-245453 B2 | 28 November 2013 |
| | | BR PI0710777 A2 | 10 January 2012 |
| | | CA 2650488 A1 | 08 November 2007 |
| | | CN 101484574 A | 15 July 2009 |
| | | EP 2018418 A1 | 28 January 2009 |
| | | EP 2018418 B1 | 10 December 2014 |
| | | IL 194894 B | 29 August 2013 |
| | | JP 2009-535025 A | 01 October 2009 |
| | | JP 2014-097068 A | 29 May 2014 |
| | | MX 2008013885 A | 11 February 2009 |
| | | US 2010-0048411 A1 | 25 February 2010 |
| CN 102516437 A | 27 June 2012 | CN 102516437 B | 21 May 2014 |
| KR 10-2023-0049009 A | 12 April 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020240067364
- KR 1020250067201